# EUROPEAN PATENT APPLICATION

(11) **EP 4 596 690 A1**
(43) Date of publication of application: **06.08.2025**
(21) Application number: 23869655.3
(22) Date of filing: 19.05.2023
(51) Int. Cl.: C12N 11/08, C12N 9/10, C12N 9/04, C12P 7/22, C12P 17/12, C12P 19/40

(54) **ENZYME IMMOBILIZATION CARRIER AND PREPARATION METHOD THEREFOR, IMMOBILIZED ENZYME AND PREPARATION METHOD THEREFOR, AND USE THEREOF**

(30) Priority: 27.09.2022 CN 202211182935
(71) Applicant: Asymchem Laboratories (Fuxin) Co., Ltd., Fuxin, Liaoning 123000 (CN)
(72) Inventor: HONG, Hao, Morrisville North Carolina 27560 (US); JAMES, Gage, Morrisville North Carolina 27560 (US); XIAO, Yi, Fuxin, Liaoning 123000 (CN); ZHANG, Na, Fuxin, Liaoning 123000 (CN); PAN, Long, Fuxin, Liaoning 123000 (CN); MA, Liteng, Fuxin, Liaoning 123000 (CN); CUI, Yuxia, Fuxin, Liaoning 123000 (CN); HOU, Bingyang, Fuxin, Liaoning 123000 (CN)
(74) Representative: Zacco GmbH
(86) International application number: PCT/CN2023/095332
(87) International publication number: WO 2024/066402

(57) **Abstract**

Provided are an enzyme immobilization carrier and a preparation method therefor, an immobilized enzyme and a preparation method therefor, and use thereof. The enzyme immobilization carrier includes a resin matrix and a divinyl sulfone group modified on the resin matrix by means of a covalent bond. **In** order to realize the directed immobilization of an enzyme, the divinyl sulfone group is loaded on the resin matrix by means of the covalent bond. **In a** subsequent enzyme immobilization reaction, by using specific binding of the divinyl sulfone group to a histidine tag, the divinyl sulfone group in the enzyme immobilization carrier is connected to a specific amino acid residue in an enzyme molecule by means of a covalent bond, without reacting with an active group in the enzyme molecule, thereby effectively avoiding excessive influence of the enzyme immobilization carrier on enzyme conformation, and thus retaining a high catalytic activity of the enzyme. Compared with a traditional metal chelating type resin-enzyme molecule binding mode, the present disclosure shows better immobility.

## Description

The present application is based upon and claims the benefit of priority from Chinese Application No. 202211182935.7 filed on 27 September 2022, the disclosure of which is incorporated herein by reference in its entirety.

### TECHNICAL FIELD

The present application relates to the field of biocatalysis, and in particular to an enzyme immobilization carrier and a preparation method therefor, an immobilized enzyme and a preparation method therefor and use thereof.

### BACKGROUND

Immobilizing an enzyme refers to combining a free enzyme with a specific carrier material through a physical or chemical method. The method includes adsorption method, covalent binding method, embedding method, and cross-linking method. Among them, covalent binding method for immobilizing an enzyme has become the most widely used method in industry because of its strong binding between the enzyme and a carrier and good operational stability.

The covalent binding method uses amino, carboxyl, thiol, hydroxyl, etc. in an enzyme protein to form a covalent bond with an organic group of a carrier, so that the enzyme is immobilized on the carrier. The method generally requires the carrier to be chemically activated to obtain a reactive group in advance, for example, an amino-type carrier is selected to be activated in advance with glutaraldehyde, and then the carrier is coupled with a corresponding group in the enzyme molecule. The reaction conditions for covalent binding are relatively violent, and are non-selective in reaction with the groups of the enzyme molecule, generally resulting in a change of the high-order structure of the enzyme molecule and thus a loss of activity. If a group associated with the active center of the enzyme is included in the covalent conjugation process, the activity of the enzyme is reduced more severely, or even the enzyme is inactivated.

Conventional enzyme carriers, such as the most widely used amino-type enzyme carriers, are generally immobilized by means of glutaraldehyde pre-activation. Due to the randomness of the binding of the carriers to groups on enzyme molecules, this method often leads to drastic changes in the enzyme conformation and thus lower catalytic activity. Metal chelating affinity carriers, while having little effect on the enzyme conformation, often lead to poor handling stability due to weak binding forces of coordinating bonds. The described two types of commonly used enzyme carriers cannot meet requirements of practical applications.

### SUMMARY

The main object of the present application is to provide an enzyme immobilization carrier and a preparation method therefor, an immobilized enzyme and a preparation method therefor and use thereof, so as to solve the problem in the prior art that the enzyme immobilization carrier is not selective and easily reacts with an active group of an enzyme during an immobilization reaction, resulting in a decrease in the activity of the enzyme.

In order to achieve the described object, according to one aspect of the present application, provided is an enzyme immobilization carrier including a resin matrix and a divinyl sulfone group covalently modified on the resin matrix.

Further, the resin matrix is an amino resin matrix and/or a hydroxyl resin matrix, and preferably, a content of functional groups in the resin matrix is 0.05mmol/g to 2mmol/g; and preferably, the resin matrix is one or more selected from brands LX-1000HA resin, ECR8409 resin, ESR-1 resin, and Sepharose 6FF resin.

In order to achieve the described object, according to one aspect of the present application, provided is a method for preparing the described enzyme immobilization carrier. The preparation method including: mixing a resin matrix and bis(vinylsulfonyl)methane, and performing an activation reaction to obtain an activated carrier; and performing a solid-liquid separation on the activated carrier, and cleaning the obtained solid to obtain the enzyme immobilization carrier.

Further, a temperature of the activation reaction is 0°C to 30°C, a time of the activation reaction is preferably 6h to 18h, a solvent used in the activation reaction is preferably a polar organic solvent, and more preferably the solvent used in the activation reaction is selected from one or more of dichloromethane, acetonitrile and isopropanol.

Further, a weight ratio of the resin matrix to bis(vinylsulfonyl)methane is 0.4:1 to 10:1.

According to another aspect of the present application, provided is an immobilized enzyme. The immobilized enzyme including the described enzyme immobilization carrier and a target enzyme covalently immobilized on the enzyme immobilization carrier.

Further, the target enzyme is one or more selected from ketone reductases, transaminases, and nucleases, and preferably, the nuclease is purine nucleoside phosphorylase.

According to another aspect of the present application, provided is a preparation method for the described immobilized enzyme. The preparation method including: mixing an enzyme immobilization carrier, a target enzyme, and a phosphate buffer solution, and performing an immobilization reaction to obtain an immobilized enzyme.

Further, a concentration of the phosphate buffer solution is 10mmol/L to 50mmol/L, and the concentration of the phosphate buffer solution is preferably 20mmol/L; a pH of the phosphate buffer solution is preferably 6 to 8, and the pH of the phosphate buffer solution is preferably 7.

Further, a weight ratio of the enzyme immobilization carrier to the target enzyme is 2:1 to 25:1.

Further, the preparation method including: mixing a target enzyme with a phosphate buffer solution to obtain an enzyme solution; mixing the enzyme solution with an enzyme immobilization carrier and performing an immobilization reaction to obtain an immobilized enzyme; and preferably, a concentration of the enzyme solution is 1mg/mL to 100mg/mL.

Further, the immobilization reaction is performed in a shaker, a rotating speed of the shaker is preferably 100rpm to 400rpm, a time of the immobilization reaction is preferably greater than or equal to 3h, a time of the immobilization reaction is preferably 3h to 24h, and a temperature of the immobilization reaction is preferably 20°C to 30°C.

According to another aspect of the present application, provided is a method for catalyzing a reduction reaction of a substrate. The method uses the described immobilized enzyme to catalyze a reduction reaction, and preferably, the described substrate is one or more selected from acetophenone, N-t-butoxycarbonyl-3-piperidone or thymidine.

By applying the technical solution of the present application, in order to achieve the targeted immobilization of an enzyme in the present application, a divinyl sulfone group is covalently loaded on a resin matrix on the basis of the design of reaction priority, and the covalent bond makes the divinyl sulfone group and the resin matrix more firmly bonded. In a subsequent enzyme immobilization reaction, the specific binding of the divinyl sulfone group to a histidine tag can be used to covalently bind the divinyl sulfone group in the enzyme immobilization carrier to a specific amino acid residue in the enzyme molecule without reacting with an active group in the enzyme molecule, thereby effectively avoiding the excessive influence of the enzyme immobilization carrier on the conformation of the enzyme, and further retaining the high catalytic activity of the enzyme. Moreover, histidine in the enzyme molecule is covalently connected to the divinyl sulfone group, and the strong binding force of the covalent bond can make the binding between the enzyme molecule and the enzyme immobilization carrier stronger, so that the immobilized enzyme obtains good operational stability and ion tolerance.

### DETAILED DESCRIPTION OF THE EMBODIMENTS

It is important to note that the examples of the present disclosure and the characteristics in the examples can be combined under the condition of no conflicts. The present disclosure will be described below in detail with reference to the examples.

As analyzed in the background art, there is a problem in the prior art that an enzyme immobilization carrier is not selective, and reacts easily with an active group of an enzyme during an immobilization reaction, resulting in a decrease in the activity of the enzyme. In order to solve this problem, the present application provides an enzyme immobilization carrier and a preparation method therefor, an immobilized enzyme and a preparation method therefor and use thereof.

In a typical embodiment of the present application, provided is an enzyme immobilization carrier, comprising a resin matrix and a divinyl sulfone group covalently loaded on the resin carrier.

The inventors found through experiments that a vinyl sulfone group is specific to a histidine tag reaction of an enzyme, and the reaction priority is higher than that of active groups such as amino and hydroxyl on an enzyme molecule. Therefore, in order to achieve the targeted immobilization of an enzyme in the present application, on the basis of the design of the reaction priority, a divinyl sulfone group is covalently loaded on the resin matrix, and the covalent bond binding makes the binding between the divinyl sulfone group and the resin matrix stronger. In a subsequent enzyme immobilization reaction, the specific binding of the divinyl sulfone group to the histidine tag can be used to covalently bind the divinyl sulfone group in the enzyme immobilization carrier to a specific amino acid residue in an enzyme molecule without reacting with an active group in the enzyme molecule, thereby effectively avoiding the excessive influence of the enzyme immobilization carrier on the conformation of the enzyme, and further retaining the high catalytic activity of the enzyme. Moreover, histidine in the enzyme molecule is covalently connected to the divinyl sulfone group, and the strong binding force of the covalent bond can make the binding between the enzyme molecule and the enzyme immobilization carrier stronger, so that the immobilized enzyme obtains good operational stability and ion tolerance.

In some embodiments, in order to obtain the binding stability of the resin matrix to the vinyl sulfone group, the resin matrix is preferably an amino resin matrix and/or a hydroxyl resin matrix, and a content of functional groups in the resin matrix is preferably 0.05mmol/g to 2mmol/g; preferably, the resin matrix includes, but is not limited to, one or more of LX-1000HA resin from Sunresin, Purolite ECR8409, ESR-1 synthesized by Nankai, and Cytiva Sepharose 6FF. These resins have high reaction activity and can be easily to modified, and have good biocompatibility per se, as well as little effect on enzyme activity.

In another typical embodiment of the present application, provided is a preparation method for the described enzyme immobilization carrier. The preparation method including: mixing a resin matrix and bis(vinylsulfonyl)methane, and performing an activation reaction to obtain an activated carrier; and performing a solid-liquid separation on the activated carrier, and cleaning the obtained solid to obtain the enzyme immobilization carrier.

The present application uses bis(vinylsulfonyl)methane to activate the resin matrix. The preparation method is simple, the conditions are mild, no other reagent or catalyst needs to be added during the reaction, and bis(vinylsulfonyl)methane is low in toxicity, safe and environmentally friendly, and has mild modification conditions, and can be widely applied to industrial production. The enzyme immobilization carrier prepared by the preparation method can effectively avoid the excessive influence of the enzyme immobilization carrier on the conformation of the enzyme in the subsequent enzyme immobilization reaction, thereby retaining the high catalytic activity of the enzyme, and improving the operational stability and ion tolerance of the immobilized enzyme at the same time.

Taking an amino resin matrix as an example, the preparation process of the described enzyme immobilization carrier is as follows:

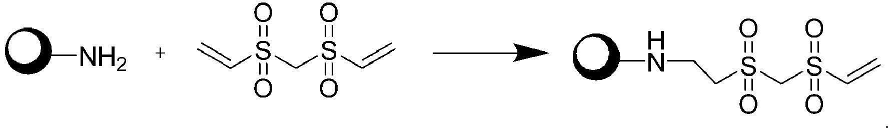

The conditions of the activation reaction are not particularly limited in the present application, and the activation conditions commonly used in the art can be applied in the present application. In order to fully activate the resin matrix and avoid damage to the structure thereof, in some embodiments, a temperature of the activation reaction is 0°C to 30°C, and preferably, a time of the activation reaction is 6h to 18h, and preferably, a solvent used in the activation reaction is a polar organic solvent, and more preferably, the solvent used in the activation reaction includes, but is not limited to, one or more of dichloromethane, acetonitrile, and isopropanol.

The solid-liquid separation can be a method commonly used in the art, for example, filtration. To remove unreacted small molecules and impurities, in some embodiments, the solvent employed for the cleaning is acetonitrile. The enzyme immobilization carrier is dried, for example, suction-dried, after washing, for the convenience of subsequent use.

In order to fully activate the resin matrix, in some embodiments, a weight ratio of the resin matrix to bis(vinylsulfonyl)methane is 0.4:1 to 10:1, and preferably 0.5:1 to 2:1. If excess bis(vinylsulfonyl)methane is used, it will cause unnecessary reagent waste and increase the modification costs; and excess bis(vinylsulfonyl)methane will cause them not be consumed by some groups or lead to more side reactions, that is, one molecule of bis(vinylsulfonyl)methane reacts with two molecules of primary amino groups. If an insufficient amount of bis(vinylsulfonyl)methane is used, the modification sites will be reduced, resulting in a low amount of enzyme protein loading subsequently, and further resulting in decreased catalytic activity.

In some embodiments, the resin matrix is dried before the activation reaction is performed.

In another typical embodiment of the present application, provided is an immobilized enzyme, including the described enzyme immobilization carrier and a target enzyme covalently immobilized on the enzyme immobilization carrier. The immobilized enzyme including the described enzyme immobilization carrier has good operational stability and high catalytic activity, and can be used as a catalyst repeatedly in a catalytic reaction.

The target enzyme is not particularly limited in the present application, and enzyme molecules commonly used in the art can be applied in the present application. In some embodiments, in order to obtain higher catalytic activity, preferably, the target enzyme includes, but is not limited to, one or more of ketoreductase (KRED), transaminase, and nuclease, and preferably, the nuclease is a purine nucleoside phosphorylase.

In another typical embodiment of the present application, provided is a method for preparing the described immobilized enzyme. The preparation method including: mixing an enzyme immobilization carrier, a target enzyme, and a phosphate buffer solution, and performing an immobilization reaction to obtain an immobilized enzyme.

The preparation method of the present application is simple and has mild conditions. The immobilized enzyme prepared by the preparation method of the present application has good operational stability and high catalytic activity, and can also be reused.

In order to buffer pH value, provide a mild suitable environment for the immobilization reaction, and avoid the decrease of the activity of the target enzyme, in some embodiments, a concentration of the phosphate buffer solution (PBS) is 10mmol/L to 50mmol/L, preferably, the concentration of the phosphate buffer solution is 20mmol/L; and preferably, a pH of the phosphate buffer solution is 6 to 8, and preferably, the pH of the phosphate buffer solution is 7. The amount of the phosphate buffer solution has small influence on the reaction, as long as the pH value can be adjusted within the range above.

In order to stabilize the binding between the enzyme and the enzyme immobilization carrier and avoid decrease in the activity of the target enzyme, in some embodiments, a weight ratio of the enzyme immobilization carrier to the target enzyme is 2:1 to 25:1, and preferably 2:1 to 20:1. From the perspective of economic costs, the target enzyme content should not be too high. If the target enzyme content is too low, the activity of the immobilized enzyme will be reduced.

In some embodiments, the preparation method including: mixing a target enzyme and a phosphate buffer solution to obtain an enzyme solution; mixing the enzyme solution with an enzyme immobilization carrier and performing an immobilization reaction to obtain an immobilized enzyme. Mixing the target enzyme and the buffer solution firstly to prepare an enzyme solution can make the target enzyme better exert activity, and then mixing and reacting the enzyme solution with the enzyme immobilization carrier can make the binding between the target enzyme and the enzyme immobilization carrier more stable and the activity of the target enzyme be better exerted.

**In** order to make the target enzyme fully active, the formulated enzyme solution is preferably at a concentration of 1mg/mL to 100mg/mL. If the concentration of the enzyme solution is too low, the immobilization of non-directional enzyme molecules will be increased, and the active yield of the enzyme is reduced; and if the concentration of the enzyme solution is too high, the binding between the enzyme molecules and the carrier will be affected, thereby reducing the catalytic activity.

The conditions of the immobilization reaction are not particularly limited in the present application, and the reaction conditions commonly used in the art can be applied in the present application. **In** some embodiments, in order to improve the reaction efficiency and sufficiently immobilize the target enzyme and the enzyme immobilization carrier, the immobilization reaction is performed in a shaker, preferably, a rotating speed of the shaker is 100rpm to 400 rpm, a time of the immobilization reaction is preferably greater than or equal to 3h, preferably, the immobilization reaction time is 3h to 24h, and preferably, a temperature of the immobilization reaction is 20°C to 30°C.

**In** some embodiments, in order to create a comfortable environment, before the immobilization reaction is performed, the enzyme immobilization carrier is replaced with a phosphate buffer solution at least once and preferably three times.

**In** a typical embodiment of the present application, provided is a method for catalyzing a reduction reaction of a substrate, using the described immobilized enzyme to catalyze a reduction reaction. Preferably, the substrate includes, but is not limited to, one or more of acetophenone, N-t-butoxycarbonyl-3-piperidone or thymidine.

**In** the present application, different immobilized enzymes are synthesized according to the types of target enzymes, and these immobilized enzymes have relatively high catalytic activity, and can be used as a catalyst for catalyzing specific reactions. When a reaction is catalyzed, a kit having the described immobilized enzyme can stably catalyze the reaction, and it can keep integrity without being broken or degraded after the reaction, and thus can be repeatedly used.

The present disclosure will be further described in detail with reference to the following examples, which should not be construed as limiting the scope of protection of the present disclosure.

### Example 1

(1) Preparation of a novel affinity carrier LX-1000HA-MDVS containing divinyl sulfone group:
   4g of dry LX-1000HA was weighed, 30mL of dichloromethane and 15mL of isopropanol were added thereto, the mixture was stirred at room temperature for 0.5h, then 2g of bis(vinylsulfonyl)methane (MDVS) was added, and the reaction was continued at room temperature for 6h; and after the reaction was completed, the resin was filtered, washed with acetonitrile in an ice bath three times, and suction-dried to obtain the prepared carrier LX-1000HA-MDVS.
(2) LX-1000HA-MDVS immobilized KRED: 1mL of a crude enzyme solution of KRED was added into 3mL of 20mmol/L PBS buffer solution (pH 7.0) to formulate an enzyme solution with a concentration of 30mg/mL. The prepared LX-1000HA-MDVS was replaced with the 20mmol/L phosphate buffer solution with pH 7.0 three times, and 1g of the carrier LX-1000HA-MDVS was weighed and dispersed in 4mL of the formulated enzyme solution. The immobilized system was placed in a shaker at 20°C and immobilized at 120rpm for 3h, and then the carrier was filtered, and washed three times with a buffer solution to obtain the immobilized KRED.

### Example 2

(1) A procedure for preparing a novel affinity carrier containing divinyl sulfone group was as follows:
   4g of dry LX-1000HA was weighed, 30mL of dichloromethane and 15mL of isopropanol were added, the mixture was stirred at 0°C for 0.5h, and then 2g of bis(vinylsulfonyl)methane (MDVS) was added at 0°C, and the reaction was continued at room temperature for 6h; and after the reaction was completed, the resin of the carrier was filtered, washed with acetonitrile in an ice bath three times, and suction-dried to obtain the prepared carrier LX-1000HA-MDVS-2.
(2) The process for preparing the immobilized enzyme was the same as that in Example 1.

### Example 3

The difference from Example 1 was that: in step (1), the LX-1000HA resin was replaced with ECR8409 resin to obtain carrier LX-1000HA-MDVS-3.

### Example 4

The difference from Example 1 was that: in step (1), 1g of dry LX-1000HA was used to obtain carrier LX-1000HA-MDVS-4.

### Example 5

The difference from Example 1 was that: in step (1), 20g of dry LX-1000HA was used to obtain carrier LX-1000HA-MDVS-5.

### Example 6

The difference from Example 1 was that: in step (1), 0.8g of dry LX-1000HA was used to obtain carrier LX-1000HA-MDVS-6.

### Example 7

(1) The process for preparing a novel affinity carrier containing divinyl sulfone group was the same as that in Example 1;
(2) LX-1000HA-MDVS immobilized KRED: a crude enzyme solution of KRED with a protein concentration of 0.12g/mL was added into a PBS buffer solution of 20mmol/L with a pH of 7.0 to formulate an enzyme solution with a concentration of 100mg/mL. The prepared LX-1000HA-MDVS was replaced with the 20mmol/L phosphate buffer solution with pH 7.0 three times, and 1g of the carrier LX-1000HA-MDVS was weighed and dispersed in 4mL of the formulated enzyme solution. The immobilized system was placed in a shaker at 20°C and immobilized at 120rpm for 3h, and then the carrier was filtered, and washed three times with a buffer solution to obtain the immobilized KRED.

### Example 8

The difference from Example 1 was that: in step (2), the amounts of the KRED crude enzyme solution and PBS buffer solution were adjusted so that the concentration of the enzyme solution was 10mg/mL.

### Example 9

The difference from Example 1 was that: in step (2), the amounts of the KRED crude enzyme solution and PBS buffer solution were adjusted so that the concentration of the enzyme solution was 120mg/mL.

### Comparative Example 1

(1) Preparation of glutaraldehyde-activated LX-1000HA carrier: 1g of LX-1000HA based on wet weight was added into 4mL of a 2% (w/v) glutaraldehyde solution, and the glutaraldehyde solution was prepared using the 20mmol/L phosphate buffer solution with a pH of 7.0. Following activation for 1h at 30°C in a shaker, the carrier was filtered and washed three times with 0.1mol/L phosphate buffer solution with pH 7.0 to obtain the glutardialdehyde-activated amino carrier LX-1000HA-GA.
(2) LX-1000HA-GA immobilized KRED: 1mL of KRED crude enzyme solution was added to 3mL of the 20mmol/L PBS buffer solution with pH 7.0 to formulate an enzyme solution with a concentration of 30mg/mL. 1g of carrier LX-1000HA-GA was dispersed in 4mL of the formulated enzyme solution. The immobilized system was placed in a shaker at 20°C and immobilized at 120rpm for 18h. Subsequently, the carrier was filtered and washed three times with a buffer solution to obtain the immobilized KRED.

### Comparative Example 2

IMAC Ni immobilized KRED: 1mL of the KRED crude enzyme solution was added to 3mL of the 20mmol/L PBS buffer solution with pH 7.0, wherein the buffer solution further comprises 50mmol/L imidazole and 150mmol/L NaCl, to formulate an enzyme solution with a concentration of 30mg/mL. 1g of carrier IMAC Ni, which was a commercial carrier, was weighed and dispersed in 4mL of the formulated enzyme solution. The system was subsequently placed in a shaker at 20°C and immobilized at 120rpm for 3h, and then the carrier was filtered and washed three times with the buffer solution to obtain immobilized KRED.

### Determination of different carrier protein loading amounts:

After the supernatant and the washing solution of the immobilized enzyme in each of the described Examples and Comparative Examples were combined respectively, the content of the unimmobilized protein was measured with Bradford, and then the protein loading amount of the carrier was calculated. The enzyme protein loading amounts of the three carriers LX-1000HA-MDVS, LX-1000HA-GA and IMAC Ni were determined to be 48mg/g, 45mg/g, and 38mg/g, respectively.

### Catalytic performance measurement of the immobilized KRED:

The reaction catalyzed by the immobilized KRED was as follows:

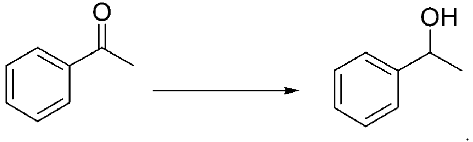

0.1g of acetophenone (CAS: 98-86-2) was weighed, 0.5mL of isopropanol, 3mL of 0.1mol/L phosphate buffer solution 7.0, 10mg of NAD⁺, and 50mg of the immobilized enzyme prepared in the described Examples and Comparative Examples were added. The reaction was then placed in a shaker at 30°C at 200rpm for 18h. LX-1000HA-MDVS, LX-1000HA-GA, and IMAC Ni immobilized KRED corresponded to substrate conversion rates of 90%, 52%, and 91%, respectively. At a lower amount of immobilized enzyme, the conversion rate of immobilized enzyme by LX-1000HA-GA was the lowest. The reasons for this phenomenon were as follows: firstly, the same mass of immobilized enzyme LX-1000HA-GA contained more heteroproteins, in other words, the specific activity was poorer; and secondly, non-selective covalent binding of glutaraldehyde caused sharp changes in enzyme conformation, and it was difficult to obtain high retention of catalytic activity.

### Operational stability assay for the immobilized KRED:

The test method for the catalytic performance of the immobilized enzyme was the same as above except that the amount of immobilization was increased to 100mg. After the end of each reaction, the immobilized enzyme was filtered and washed three times with a buffer solution. The immobilized enzyme was subsequently added to the next reaction cycle. The reusability of the three immobilized enzymes was shown in Table 1.

**Table 1**

| | Substrate conversion (%) | | | | |
|---|---|---|---|---|---|
| | 1st | 2^{nd} | 3^{rd} | 4^{th} | 5^{th} |
| Example 1 (LX-1000HA-MDVS) | 99 | 99 | 97 | 98 | 97 |
| Example 2 | 97 | 97 | 94 | 95 | 95 |
| Example 3 | 92 | 95 | 94 | 93 | 89 |
| Example 4 | 97 | 95 | 96 | 93 | 93 |
| Example 5 | 74 | 72 | 69 | 63 | 52 |
| Example 6 | 86 | 85 | 81 | 84 | 77 |
| Example 7 | 99 | 99 | 95 | 96 | 94 |
| Example 8 | 51 | 55 | 47 | 45 | 38 |
| Example 9 | 99 | 99 | 98 | 98 | 95 |
| Comparative Example 1 (LX-1000HA-GA) | 58 | 59 | 49 | 45 | 27 |
| Comparative Example 2 (IMAC Ni) | 99 | 99 | 83 | 75 | 52 |

### Example 10

The difference from Example 1 was that: in step (2), KRED was replaced with a His-tagged transaminase (TA), the transaminase was immobilized on LX-1000HA-MDVS, 1mL of the crude enzyme solution of KRED was replaced with 0.1g of transaminase powder, and 5mg of cofactor-pyridoxal phosphate PLP was added.

### Comparative Example 3

The difference from Comparative Example 1 was that: in step (2), KRED was replaced with a His-tagged transaminase (TA), the transaminase was immobilized on LX-1000HA-GA, the 1mL of the crude enzyme solution of KRED was replaced with 0.1g of transaminase powder, and 5mg of cofactor-pyridoxal phosphate PLP was added.

### Comparative Example 4

The difference from Comparative Example 2 was that: KRED was replaced with a His-tagged transaminase (TA), the transaminase was immobilized on IMAC Ni, 1mL of the crude enzyme solution of KRED was replaced with 0.1g of transaminases powder, and 5mg of cofactor-pyridoxal phosphate PLP was added.

Investigation on activity and reusability of immobilized transaminase: the reaction catalyzed by the immobilized TA was as follows:

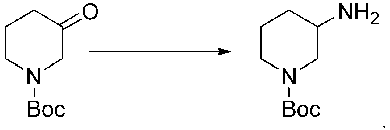

0.1g of N-t-butoxycarbonyl-3-piperidone (CAS: 98977-36-7) was weighed, 4eq ("eq" represents equivalent amount) of isopropylamine hydrochloride, 1mg of PLP, and 1mL of 0.1mol/L PBS buffer solution (pH of PBS buffer solution was 7.0) were added, followed by 100mg of the immobilized enzyme prepared in Example 10 and Comparative Examples 3-4 at 30°C. The reaction was conducted at 200rpm for 18h, and the conversion rate was measured. At the end of each reaction, the immobilized enzyme was filtered and washed three times with a buffer solution, and the immobilized enzyme was subsequently put into the next reaction cycle. The reaction data were shown in Table 2.

**Table 2**

| Immobilized enzyme carrier | Conversion rate (%) | Number of cycles |
|---|---|---|
| Example 10 (LX-1000HA-MDVS) | >92 | 10 |
| Comparative Example 3 (LX-1000HA-GA) | >90 | 10 |
| Comparative Example 4 (IMAC Ni) | >80 | 4 |

### Example 11

The difference from Example 1 was that: in step (2), KRED was replaced with a His-tagged purine nucleoside phosphorylase (PNP), and the His-tagged purine nucleoside phosphorylase was immobilized on LX-1000HA-MDVS, and 1mL of the crude enzyme solution of KRED was replaced with 1 mL of a PNP enzyme solution.

### Comparative Example 5

The difference from Comparative Example 1 was that: in step (2), KRED was replaced with a His-tagged purine nucleoside phosphorylase (PNP), and the His-tagged purine nucleoside phosphorylase was immobilized on LX-1000HA-GA, and 1mL of the crude enzyme solution of KRED was replaced with 1mL of the PNP enzyme solution.

### Comparative Example 6

The difference from Comparative Example 2 was that: KRED was replaced with a His-tagged purine nucleoside phosphorylase (PNP), and 1mL of the crude enzyme solution of KRED was replaced with 1 mL of the PNP enzyme solution.

Investigation on activity and reusability of immobilized PNP:

The reaction catalyzed by the immobilized PNP was as follows:

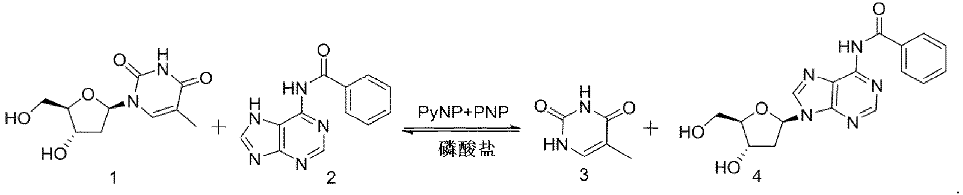

0.15g of thymidine (CAS: 50-89-5) and 0.095 g of N6-benzoyladenine (CAS: 4005-49-6) were weighed, 1mL of 2mmol/L KPB 7.5, 0.02mL of pyrimidine nucleoside phosphorylase (PyNP) and 50mg of the PNP immobilized enzyme prepared in Example 11 and Comparative Examples 5-6 were added. The reaction was conducted at 60°C in a shaker at 200rpm for 16h, and the conversion rate was measured. At the end of each reaction, the immobilized enzyme was filtered and washed three times with a buffer solution, and the immobilized enzyme was subsequently put into the next reaction cycle. The reaction data was shown in Table 3.

**Table 3**

| Immobilized enzyme carrier | Conversion rate (%) of N6-benzoyladenine | Number of cycles |
|---|---|---|
| Example 11 (LX-1000HA-MDVS) | >85 | 10 |
| Comparative Example 5 (LX-1000HA-GA) | >27 | 1 |
| Comparative Example 6 (IMAC Ni) | >82 | 5 |

From the description above, it can be determined that the described examples of the present application achieve the following technical effects: in the present application, on the basis of the design of the reaction priority, the bivinyl sulfone group is covalently loaded on the resin matrix, and the covalent bond makes the binding between the bivinyl sulfone group and the resin matrix stronger. **In** the subsequent enzyme immobilization reaction, the specific binding of the bivinyl sulfone group to the histidine tag can be used to covalently bind the bivinyl carboxyl group in the enzyme immobilization carrier to a specific amino acid residue in the enzyme molecule without reacting with an active group in the enzyme molecule, thereby effectively avoiding the excessive influence of the enzyme immobilization carrier on the conformation of the enzyme, and further retaining the high catalytic activity of the enzyme. Moreover, histidine in the enzyme molecule is covalently linked to the bivinyl sulfone group, and the strong binding force of the covalent bond can make the binding between the enzyme molecule and the enzyme immobilization carrier more firm, so that the immobilized enzyme obtains good operational stability and ionic tolerance. **In** addition, in the present application, bis(vinylsulfonyl)methane is used to modify the resin matrix, and the reagent is easy to obtain, low in toxicity, safe and environmentally friendly, and can be widely applied to industrial production.

The foregoing descriptions are merely exemplary embodiments of the present application, but are not intended to limit the present application. For a person skilled in the art, the present application may have various modifications and variations. Any modifications, equivalent replacements, improvements and the like made within the spirit and principle of the present application shall fall within the scope of protection of the present application.

## Claims

1. An enzyme immobilization carrier, wherein the enzyme immobilization carrier comprising a resin matrix and a divinyl sulfone group covalently modified on the resin matrix.

2. The enzyme immobilization carrier according to claim 1, wherein the resin matrix is an amino type resin matrix and/or a hydroxyl type resin matrix, and a content of functional groups in the resin matrix is preferably 0.05mmol/g to 2mmol/g; and preferably, the resin matrix is selected from one or more of brands LX-1000HA resin, ECR8409 resin, ESR-1 resin, and Sepharose 6FF resin.

3. A preparation method for the enzyme immobilization carrier according to claim 1 or 2, wherein the preparation method including:
mixing the resin matrix with bis(vinylsulfonyl)methane and performing an activation reaction to obtain an activated carrier;
performing a solid-liquid separation on the activated carrier, and cleaning the obtained solid to obtain the enzyme immobilization carrier.

4. The preparation method for the enzyme immobilization carrier according to claim 3, wherein a temperature of the activation reaction is 0°C to 30°C, a time of the activation reaction is preferably 6h to 18h, a solvent used in the activation reaction is preferably a polar organic solvent, and more preferably the solvent used in the activation reaction is selected from one or more of dichloromethane, acetonitrile and isopropanol.

5. The preparation method for the enzyme immobilization carrier according to claim 3, wherein a weight ratio of the resin matrix to the bis(vinylsulfonyl)methane is 0.4:1 to 10:1.

6. An immobilized enzyme, wherein the immobilized enzyme comprising the enzyme immobilization carrier according to claim 1 or 2 and a target enzyme covalently fixed on the enzyme immobilization carrier.

7. The immobilized enzyme according to claim 6, wherein the target enzyme is selected from one or more of ketoreductase, transaminase and nuclease, and the nuclease is preferably purine nucleoside phosphorylase.

8. A preparation method for the immobilized enzyme according to claim 6 or 7, wherein the preparation method including:
mixing the enzyme immobilization carrier, the target enzyme and a phosphate buffer and performing an immobilization reaction to obtain the immobilized enzyme.

9. The preparation method for the immobilized enzyme according to claim 8, wherein a concentration of the phosphate buffer is 10mmol/L to 50mmol/L, preferably 20mmol/L; and a pH of the phosphate buffer is 6 to 8, preferably 7.

10. The preparation method for the immobilized enzyme according to claim 8 or 9, wherein a weight ratio of the enzyme immobilization carrier to the target enzyme is 2: 1 to 25:1.

11. The preparation method for the immobilized enzyme according to any one of claims 8 to 10, wherein the preparation method includes:
mixing the target enzyme with the phosphate buffer to obtain an enzyme solution;
mixing the enzyme solution with the enzyme immobilization carrier and performing an immobilization reaction to obtain the immobilized enzyme; and
a concentration of the enzyme solution is preferably 1mg/mL to 100mg/mL.

12. The preparation method for the immobilized enzyme according to any one of claims 8 to 11, wherein the immobilization reaction is performed in a shaker, a rotating speed of the shaker is preferably 100rpm to 400rpm, a time of the immobilization reaction is preferably greater than or equal to 3h, a time of the immobilization reaction is preferably 3h to 24h, and a temperature of the immobilization reaction is preferably 20°C to 30°C.

13. A method for catalyzing a substrate for performing a reduction reaction, wherein the method using the immobilized enzyme according to claim 6 or 7 to catalyze the reduction reaction, and preferably the substrate is selected from one or more of acetophenone, N-tert-butoxycarbonyl-3-piperidone or thymidine.
